(19) Europäisches Patentamt
European Patent Office
Office européen des brevets

(11) Numéro de publication: **0 066 934**
**A1**

(12) **DEMANDE DE BREVET EUROPEEN**

(21) Numéro de dépôt: **82200687.0**

(22) Date de dépôt: **04.06.82**

(51) Int. Cl.³: **C 07 D 405/12**, A 61 K 31/415

(30) Priorité: **09.06.81 LU 83422**

(43) Date de publication de la demande: **15.12.82 Bulletin 82/50**

(84) Etats contractants désignés: **AT BE CH DE FR GB IT LI NL SE**

(71) Demandeur: **"PHARLYSE", Boulevard Royal, 2, Luxembourg (LU)**

(72) Inventeur: **Deboeck, Arthur Marie, 61 A Steenweg op Edingen, B-1540 Herne (BE)**
Inventeur: **Fossion, Jacques Jean, 18, rue du Cours d'Eau, B-1428 Lillois (BE)**
Inventeur: **Smolders, Robert René, 34 Avenue de l'Arbalète, B-1170 Bruxelles (BE)**

(74) Mandataire: **Schmitz, Yvon et al, Bureau Gevers S.A. 7, rue de Livourne Bte 1, B-1050 Bruxelles (BE)**

(54) **Sels de nitrofurantoine, leur préparation et leur utilisation.**

(57) Sels de nitrofurantoïne hydrosolubles, constitués par les produits de réaction de la nitrofurantoïne et au moins d'un acide aminé basique, l'acide aminé basique étant de préférence choisi dans le groupe formé par l'arginine, la lysine, l'histidine, l'ornithine et la glycine.

EP 0 066 934 A1

ACTORUM AG

"Sels de nitrofurantoïne, leur préparation et leur utilisation".

La présente invention est relative à des sels pharmaceutiquement utilisables, solubles dans l'eau de la nitrofurantoïne.

La nitrofurantoïne ou N-(5-nitro-2-furfurylidène)-1-aminohydantoïne est un médicament, bien connu, utilisé notamment comme agent antiseptique des voies urinaires. Il présente toutefois l'inconvénient d'être très peu soluble dans l'eau (19 mg/100 ml à 25°C), ce qui gêne son utilisation dans la plupart des formes pharmaceutiques. Afin de pallier à cet inconvénient, différentes formes cristallines ont été développées avec peu de succès. Des sels de métaux alcalins de nitrofurantoïne ont également été réalisés mais ceux-ci, bien que solubles, présentent l'inconvénient d'apporter avec eux, des ions alcalins qui sont souvent contre-indiqués, notamment lors des traitements au long cours chez les sujets soumis à un régime désodé.

La présente invention a pour but de remédier à ces inconvénients, et de mettre au point un sel de nitrofurantoïne fortement soluble dans l'eau, qui possède une meilleure biodisponibilité , c'est-à-dire une résorption plus importante pour une même dose de substance active, et/ou une meilleure tolérance notamment dans le tractus gastro-

intestinal, que la nitrofurantoïne et ses sels de métaux alcalins.

A cet effet, suivant l'invention, le sel est constitué par le produit de réaction de la nitrofurantoïne et au moins d'un acide aminé basique.

Avantageusement, le sel contient environ 1 à 5 moles, et de préférence environ 1 à 2 moles d'acide aminé basique par mole de nitrofurantoïne.

Le ou les acides aminés basiques utilisés peuvent être naturels ou non comme par exemple, l'arginine, la lysine, l'histidine, l'ornithine et la glycine. Les acides aminés basiques selon la présente invention peuvent renfermer un ou plusieurs centres asymétriques et peuvent donc exister sous les formes isomères optiquement actives. Il est bien entendu que l'invention couvre les deux formes épimères, telles que les formes lévogyre et dextrogyre, ainsi que leur mélange.

Des exemples d'acides aminés basiques lévogyres et dextrogyres sont les D- et L-lysines et les D- et L-arginines.

Le sel de nitrofurantoïne peut également, en fonction du mode de préparation, contenir une ou plusieurs molécules de solvant de cristallisation. De façon tout à fait inattendue, cette ou ces molécules de solvant de cristallisation augmente la stabilité du sel dans le temps et diminue fortement son hygroscopicité, ce qui en facilite fortement son utilisation. Des exemples de solvant de cristallisation sont les alcools, polyglycols, glycols.

L'invention concerne également la préparation de ces sels de nitrofurantoïne.

Selon une première façon de procéder, la nitrofurantoïne est ajoutée à une solution aqueuse contenant le ou les acides aminés basiques, l'eau étant ensuite séparée du mélange réactionnel ainsi obtenu par des méthodes de séparation appropriées, telles que par évaporation ou lyophilisation.

Une autre façon de procéder consiste à ajouter une solution aqueuse ou hydroorganique contenant le ou les acides aminés basiques à une solution organique contenant la nitrofurantoïne et à séparer le solvant du mélange réactionnel ainsi obtenu, par des méthodes de séparation appropriées, telles que par filtration, lyophilisation ou évaporation. L'ordre d'addition **peut** être inversé. Des exemples de solvants organiques utilisés pour dissoudre les acides aminés et la nitrofurantoïne sont les solvants organiques polaires, tels que les alcools, les glycols, les polyglycols, les cétones, le diméthylformamide et le diméthylsulfoxyde. On peut également utiliser des mélanges de tels solvants.

Une troisième façon de procéder consiste à ajouter une solution aqueuse ou hydroorganique contenant le ou les acides aminés basiques à une solution ou suspension organique ou hydroorganique contenant la nitrofurantoïne. Le milieu réactionnel trouble est filtré et le produit

cristallise par adjonction de solvant organique polaire. Des solvants organiques précipitants sont les alcools, polyglycols, glycols, acétates, éthers, esters. On peut également utiliser des mélanges de tels solvants.

Dans tous les cas, le traitement susdit est effectué à une température de l'ordre de -5°C à 100°C, et de préférence à une température de l'ordre de 20°C.

Une quatrième façon de procéder consiste à mélanger intimement à sec les réactifs solides, c'est-à-dire le ou les acides aminés et la nitrofurantoïne.

La nitrofurantoïne est un acide faible dont le pKa en solution aqueuse se situe aux environs de 7,2. Ainsi qu'on l'a déjà précisé précédemment, les sels se formant aisément avec les acides aminés basiques, tels que l'arginine, la lysine, l'histidine, l'ornithine et la glycine. L'obtention d'un sel parfaitement soluble dans l'eau requiert entre 1 et 5 molécules et de préférence 1 à 2 molécules d'acide aminé par molécule de nitrofurantoïne.

En fait, les sels de nitrofurantoïne de l'invention répondent à la formule suivante :

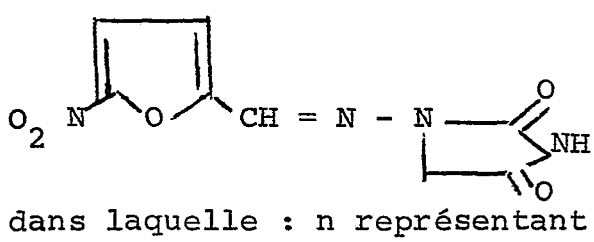

. nX . mY

dans laquelle : n représentant le nombre de molécules d'acide aminé par rapport à 1 molécule de nitrofurantoïne, est compris entre 1 et 5, et de

préférence égal à 1 ou 2, X représente le ou les acides aminés, m représentant le nombre de molécules de solvant(s) de cristallisation par rapport à 1 molécule de nitrofurantoïne, est égal à 0,1 ou 2, et Y représente le ou les solvants de cristallisation.

Les sels de nitrofurantoïne hydrosolubles de la présente invention peuvent donc être obtenus en utilisant des méthodes connues de préparation de sels, et, en particulier, par la mise en solution aqueuse ou hydroorganique d'un ou de plusieurs acides aminés, à laquelle, tout en maintenant la température comprise entre -5°C et 100°C et de préférence aux alentours de 20°C, on ajoute, sous agitation, par petites portions la quantité de nitrofurantoïne requise, éventuellement sous forme de solution organique. On notera, à cet effet, que l'on peut inverser l'ordre d'addition des réactifs mis en présence.

On élimine le solvant du mélange réactionnel par des méthodes de séparation appropriées quelconques, telles que par filtration (solution hydroorganique), par lyophilisation (solution aqueuse) ou par chauffage modéré sous vide. On obtient ainsi une poudre soluble dans l'eau qui peut être utilisée pour la préparation de formes solides (comprimés, suppositoires, tablettes, granulés, dragées) et de formes injectables. Il va de soi que les solutions de sels de nitrofurantoïne, préparés comme expliqué ci-dessous, peuvent être utilisées immédiatement sous forme injectable sans être lyophilisées au

préalable, et pour autant que leur force ionique soit acceptable ou rendue telle, de même que leur stérilité.

Ci-dessous sont rassemblés quelques exemples, non limitatifs, de préparation des composés suivant l'invention.

Exemple 1.

On refroidit à 0°C une solution de 11,68 g de lysine base dans 190 ml d'eau. A cette solution limpide, on ajoute par petites portions et sous agitation 10,47 g de nitrofurantoïne. Quelques minutes après la fin de l'addition, la solution est filtrée, congelée et lyophilisée. On obtient un produit de saveur amère instantanément soluble dans l'eau et présentant les caractéristiques suivantes : pH d'une solution à 10% dans l'eau = 8,9 - 9,5; point de fusion : 150°C avec décomposition; solubilité dans l'eau à 20°C : supérieure à 30%.

En procédant de la même façon avec l'aginine, l'histidine ou l'ornithine, on obtient les sels correspondants qui sont tous instantanément solubles dans l'eau.

Exemple 2.

A une solution de 11,68 g de lysine base dans 30 ml d'eau et 150 ml d'éthanol, maintenue à température ambiante,on ajoute, sous agitation, 14,28 g de nitrofurantoïne par petites portions. Après avoir ajouté 200 ml d'éthanol supplémentaire et après un repos de 10 minutes, on filtre le produit. Après un séchage de 24 heures à 60°C, on obtient 22,5 g (rendement de 87%) de sel de

lysine cristallisé avec une molécule d'éthanol : P.F. 160-165°C,décomposition

- teneur en nitrofurantoïne : 55,4%
- teneur en lysine : 33,9%
- teneur en éthanol : 10,7%

Exemple 3.

A une solution aqueuse de 6 ml de lysine contenant 251 mg de lysine par ml, dans laquelle on a ajouté 15 ml d'éthanol et 5 ml d'acétone, cette solution étant maintenue à une température de 5°C, on ajoute en une fois 1,18 g de nitrofurantoïne en solution dans 5 ml d'acétone et 5 ml de diméthylsulfoxyde. Après quelques minutes, le produit cristallise. On ajoute un peu d'acétone pour diluer, on filtre et on lave à l'acétone puis à l'éther de pétrole. On récupère 2,10 g (rendement de 78%)de sel de lysine soluble dans l'eau .

Exemple 4.

A une solution de 11,68 g de lysine base et de 3 g de glycine dans 140 ml d'eau maintenue à 0°C et sous agtitation, on ajoute par petites portions 10,47 g de nitrofurantoïne. Quelques minutes après la fin de l'addition, la solution limpide est filtrée. On récupère environ 25 g de produit après lyophilisation.

Exemple 5.

292 g de lysine base anhydre sont broyés intimement avec 238 g de nitrofurantoïne. La poudre ainsi obtenue est tamisée et granulée au moyen de 200 ml d'un mélange alcool-éther (50/50 en vol/vol.). Après séchage à 40°C, on

obtient un produit parfaitement soluble dans l'eau.

Les sels de nitrofurantoïne solubles dans l'eau de la présente invention exercent une activité antiseptique remarquable au niveau des voies urinaires.

Les sels à base de nitrofurantoïne de l'invention peuvent être administrés en association avec divers excipients pharmaceutiques, tels que des diluants, des gélifiants, des agents conservateurs, des émulsionnants, des édulcorants et aromatisants, etc, et cela par voie orale, parentérale ou rectale.

Pour une administration orale, on utilisera des dragées, granulés, pastilles (pellets), tablettes, capsules, comprimés , solutions, sirops, émulsions contenant des additifs ou excipients classiques en pharmacie galénique. Ces formes galéniques peuvent libérer le principe de façon normale ou programmée dans le temps.

Pour l'administration parentérale, on utilisera de l'eau stérile ou une huile d'arachide ou l'oléate d'éthyle.

Pour l'administration rectale, on utilisera des suppositoires, des capsules rectales, des solutions ou des gelées.

Le composé actif peut être administré seul ou en combinaison avec d'autres produits actifs ayant une activité similaire ou différente.

Les doses recommandées pour l'administration par la voie orale, sont par exemple de 100 mg à 3 g, avantageusement de 200 mg à 1,5 g par jour, et pour l'administration par voie

intraveineuse ou intramusculaire de 100 mg à 1g par jour.

On donne ci-après quelques exemples de formes solides contenant, comme principe actif, un des composés suivant l'invention.

Exemple 1

a) Formulation pour 1 comprimé :

| | | |
|---|---|---|
| Nitrofurantoïne (sel de lysine) | : | 90 mg |
| Lactose impalpable | : | 180 mg |
| Polyméthacrylate | : | 25 mg |
| Cellulose microcristalline | : | 84 mg |
| Polyvinylpyrrolidone réticulée | : | 20 mg |
| Stéarate de magnésium | : | 1 mg |
| Acétate d'éthyle | : | quantité suffisante |
| | | 400 mg |

b) Procédé de fabrication

1 - Granuler ensemble, au moyen d'acétate d'éthyle, le sel de lysine de la nitrofurantoïne, le lactose et le polyméthacrylate, tamiser et sécher.

2 - Tamiser à nouveau le granulé sec et y mélanger la cellulose, la polyvinylpyrrolidone et le stéarate de magnésium.

3 - Comprimer à 0,400 g par comprimé.

Exemple 2

a) Formulation pour une dose de microgranules

| | | |
|---|---|---|
| Nitrofurantoïne (sel de lysine) | : | 90 mg |
| Lactose impalpable | : | 250 mg |

| | | |
|---|---|---|
| Hydroxypropylméthylcellulose | : | 40 mg |
| Gomme laque | : | quantité suffisante |
| Dichlorométhane | : | quantité suffisante |
| Alcool éthylique | : | quantité suffisante |

b) Procédé de fabrication

1 - Granuler ensemble, au moyen de la solution d'hydroxypropylméthylcellulose dans le dichlorométhane, le sel de nitrofurantoïne et le lactose.

2 - Traiter ce granulé avant dessication, par tout moyen adéquat pour en faire des microgranules plus ou moins sphériques.

3 - Sécher les microgranules et les tamiser afin de sélectionner le diamètre souhaité.

Ensuite, éventuellement

4 - Projeter, sur les microgranules ainsi obtenus, la gomme laque en solution alcoolique.

5 - Sécher parfaitement les microgranules, les tamiser à nouveau et contrôler la cinétique de libération de la nitrofurantoïne suivant le type désiré (retard, prolongé, etc).

6 - Mettre en gélules les microgranules obtenues.

On notera qu'une libération programmée du principe actif peut être obtenue par d'autres composants que la gomme laque, comme par exemple, l'éthylcellulose, l'acétophtalate de cellulose, les polyméthacrylates. Il est aussi évident que l'emploi de ces substances est indiqué lorsque l'on veut obtenir une libération retardée ou programmée de la substance active; si une libération rapide doit être obtenue les étapes 4 et 5 peuvent être supprimées pour passer directement de l'étape 3 à l'étape 6.

Exemple 3

a) Formulation pour une dose de microgranules

| | | |
|---|---|---|
| Microgranules neutres constitués par exemple d'environ 75% de saccharose et 25% d'amidon | : | 250 mg |
| Nitrofurantoïne (sel de lysine) | : | 90 mg |
| Hydroxypropylméthylcellulose | : | 25 mg |
| Polyméthacrylate | : | quantité suffisante |
| Phtalate de diéthyle, ou autre plastifiant de faible poids moléculaire | : | quantité éventuellement suffisante |
| Dichlorométhane | : | quantité suffisante |
| Acétate d'éthyle | : | quantité suffisante |

b) Procédé de fabrication

1 - Placer les microgranules neutres dans une turbine à dragéifier, ou tout autre appareillage adéquat, et projeter sur ceux-ci une suspension de nitrofurantoïne (sel de lysine) dans la solution d'hydroxypropylméthylcellulose dans le dichlorométhane, ce qui conduit à enrober les microgranules neutres au moyen du principe

actif. Répéter éventuellement plusieurs fois cet enrobage en séchant entre chaque pulvérisation jusqu'à obtenir le titre souhaité de 90 mg de nitrofurantoïne (sel de lysine) par dose unitaire de microgranules.

Eventuellement :

2 - Projeter, sur les microgranules ainsi obtenus, la solution de polyméthacrylate dans l'acétate d'éthyle afin de former un enrobage extérieur qui assurera une libération prolongée du principe actif.

3 - Après séchage complet des microgranules, contrôler la cinétique de libération de la nitrofurantoïne (sel de lysine) .
Si une libération rapide du principe actif est souhaitée, les étapes 2 et 3 peuvent être supprimées pour passer directement à l'étape 4.

4 - Mettre en gélules les microgranules obtenus.

On notera que les microgranules obtenus dans les exemples 2 et 3 peuvent être présentés, outre la mise en gélules, sous forme de comprimés, de suspension, de suppositoires ou toute autre forme pharmaceutique jugée acceptable.

De plus, les comprimés obtenus dans l'exemple 1 peuvent être réalisés de telle sorte que la libération du principe actif soit ou bien rapide ou bien retardée et/ou prolongée. Ceci s'obtiendra, notamment, en modifiant les quantités respectives du polymère méthacylique et de la cellulose microcristalline et/ou en enrobant les comprimés ainsi qu'il est indiqué pour les microgranules de l'exemple 2.

Des études de toxicité aiguë par la voie orale sur le sel de nitrofurantoïne de l'exemple 2 ont donné les résultats suivants, exprimés en dose létale pour 50% des animaux

- Rats (2 sexes) : 1908 mg par Kg de poids corporel (soit 1050 mg en nitrofurantoïne pure)
- Souris (2 sexes) : 960 mg par Kg de poids corporel (soit 530 mg en nitrofurantoïne pure)

La toxicité de la nitrofurantoïne est connue pour donner des résultats variables suivant les caractéristiques du produit utilisé, notamment la taille des cristaux. Les chiffres obtenus avec la substance suivant l'invention montrent une résorption rapide et importante. On ne remarque pas non plus d'effet létal retardé, ce qui constitue également un élément favorable dans ce type d'étude.

REVENDICATIONS.

1. Sel de nitrofurantoïne hydrosoluble, caractérisé en ce qu'il est constitué par le produit de réaction de la nitrofurantoïne et au moins d'un acide aminé basique.

2. Sel suivant la revendication 1, caractérisé en ce que l'acide aminé basique est un acide aminé lévogyre, dextrogyre ou un mélange de tels acides.

3. Sel suivant l'une ou l'autre des revendications 1 et 2, caractérisé en ce que l'acide aminé basique est choisi dans le groupe formé par l'arginine, la lysine, l'histidine, l'ornithine et la glycine.

4. Sel suivant l'une quelconque des revendications 1 à 3, caractérisé en ce qu'il contient environ 1 à 5 moles d'acide aminé basique par mole de nitrofurantoïne.

5. Sel suivant la revendication 4, caractérisé en ce qu'il contient environ 1 à 2 moles d'acide aminé basique par mole de nitrofurantoïne.

6. Sel suivant la revendication 5, caractérisé en ce qu'il contient au moins une molécule de solvant de cristallisation.

7. Sel suivant la revendication 6, caractérisé en ce que le solvant de cristallisation est un solvant polaire, tel qu'un alcool, glycol, polyglycol, ester ou éther.

8. Procédé de préparation du sel de nitrofurantoïne hydrosoluble suivant l'une quelconque des revendications 1 à 5, caractérisé en ce

que l'on traite l'acide aminé basique en solution aqueuse avec de la nitrofurantoïne et en ce que l'on sépare l'eau du mélange réactionnel obtenu par des méthodes de séparation appropriées quelconques, telles que par évaporation ou lyophilisation.

9. Procédé de préparation du sel de nitrofurantoïne hydrosoluble suivant l'une quelconque des revendications 1 à 5, caractérisé en ce que l'on traite l'acide aminé basique en solution aqueuse ou hydroorganique avec de la nitrofurantoïne en solution ou suspension organique ou hydroorganique et en ce que l'on sépare le solvant du mélange réactionnel ainsi obtenu par des méthodes de séparation appropriées quelconques, telles que par filtration, lyophilisation ou évaporation.

10. Procédé suivant l'une ou l'autre des revendications 8 et 9, caractérisé en ce que le traitement susdit est effectué à une température comprise entre -5°C et 100°C.

11. Procédé suivant la revendication 10, caractérisé en ce que le traitement est effectué à une température de l'ordre de 20°C.

12. Procédé suivant l'une quelconque des revendications 9 à 11, caractérisé en ce que le solvant organique utilisé pour dissoudre ou disperser l'acide aminé et la nitrofurantoïne est un solvant organique polaire ou un mélange de solvants organiques polaires.

13. Procédé de préparation du sel de nitrofurantoïne suivant l'une quelconque des revendications 1 à 5, caractérisé en ce que l'on mélange intimement à sec l'acide aminé basique avec la nitrofurantoïne.

14. Composition pharmaceutique comprenant, comme produit actif, au moins un sel de nitrofurantoïne hydrosoluble suivant l'une quelconque des revendications 1 à 7, associé à au moins un excipient approprié et/ou à au moins un autre agent thérapeutique.

15. Méthode d'utilisation des sels de nitrofurantoïne suivant l'une quelconque des revendications 1 à 7 ou de la composition suivant la revendication 14, comme agent antiseptique des voies urinaires, caractérisée en ce que l'on administre des sels de la nitrofurantoïne, par voie orale, à des doses journalières de 100 mg à 3 g, et par voie intramusculaire ou intraveineuse, à des doses journalières de 100 mg à 700 mg ou plus par jour.

Office européen des brevets

# RAPPORT DE RECHERCHE EUROPEENNE

0066934

Numéro de la demande

EP 82 20 0687

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| A | FR-M- 2 646 (BOEHRINGER & SOEHNE)<br>*En entier*<br>--- | 1-15 | C 07 D 405/12<br>A 61 K 31/415 |
| A | FR-M- 1 542 (LEO INDUSTRIE CHIMICHE FARMACEUTICHE)<br>*En entier*<br>----- | 1-15 | |
| | | | **DOMAINES TECHNIQUES RECHERCHES (Int. Cl. ³)** |
| | | | C 07 D 405/00 |

Le présent rapport de recherche a été établi pour toutes les revendications

| Lieu de la recherche | Date d'achèvement de la recherche | Examinateur |
|---|---|---|
| LA HAYE | 16-08-1982 | ALLARD M.S. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503.03.82